# EUROPEAN PATENT APPLICATION

(11) **EP 1 839 665 A1**
(43) Date of publication of application: **03.10.2007**
(21) Application number: 05819798.9
(22) Date of filing: 26.12.2005
(51) Int. Cl.: A61K 31/79, A61K 9/26, A61K 33/18, A61K 47/10, A61K 47/36, A61K 47/42, A61P 1/02, A61P 17/02, A61P 31/02

(54) **INTRAORAL DISINTEGRATION TYPE SOLID PREPARATION CONTAINING POVIDONE IODINE**

(30) Priority: 27.12.2004 JP 2004375163
(71) Applicant: Kowa Company, Ltd., Nagoya-shi, Aichi 460-0003 (JP)
(72) Inventor: KANO, Yuichiro, c/o Fuji Research Lab., Fuji-shi, Shizuoka 417-8650 (JP); KUGA, Hiroaki, c/o Fuji Research Lab., Fuji-shi, Shizuoka 417-8650 (JP); MAETANI, Shigehiro, c/o Fuji Research Lab., Fuji-shi, Shizuoka 417-8650 (JP)
(74) Representative: polypatent
(86) International application number: PCT/JP2005/023701
(87) International publication number: WO 2006/070705

(57) **Abstract**

An orally disintegratable solid preparation comprising povidone-iodine as an active ingredient, which is obtained by direct powder compression of a mixture containing a granular sugar alcohol and povidone-iodine, wherein the mixture does not contain a basic ingredient and is not subjected to wet granulation. High quality solid preparation as an orally disintegratable preparation comprising povidone-iodine, which have a constant content of povidone-iodine per a single unit solid preparation and maintain stability of iodine, are provided.

## Description

### Field of the Invention

The present invention relates to an orally disintegrable solid preparation comprising povidone-iodine.

### Background Art

Povidone-iodine (Merck Index 13th edition, 2001) is a complex of polyvinylpyrrolidone as a polymer of 1-vinyl-2-pyrrolidone and iodine, and has been widely used all over the world as a safe medicament having a bactericidal action for an agent for treating bedsore, sterilization of fingers and hands, disinfection of oral cavity, pharynx and the like. A gargle which is diluted with water before use has been generally used as a povidone-iodine agent for disinfection of the oral cavity and pharynx. However, the aforementioned preparation has a fault of complicated handling because it needs to be diluted before use.

As a preparation overcoming the above fault, a spray preparation has been provided which comprises povidone-iodine dissolved in water and glycerin and added with a suitable sweetener, aromatic and the like. However, this preparation suffers from poor portability due to a bulky container, and also has a problem that the preparation may cause leak of a solution from the container. Moreover, because this spray is to be directly sprayed to pharynx, it only achieves disinfection of throat. Therefore, for disinfection of oral cavity, the spray is inconvenient because it further requires the use of a gargle.

If an orally disintegratable solid preparation comprising povidone-iodine can be provided, the aforementioned problem might possibly be solved. However, because a content of povidone-iodine per 1 unit solid preparation is very small (i.e., it is desirable to provide a solid preparation whose tablet having a unit weight of 300 to 1,000 mg contains about 5 mg of povidone-iodine), another problem arises that obtaining a constant content of povidone-iodine is difficult.

As an orally administrable solid preparation containing povidone-iodine, a tablet for treating a disease of oral cavity and throat is known which is obtained by making a core tablet with povidone-iodine, citric acid and the like, and then coating the core tablet with a material containing diclofenac sodium and hydroxypropylmethylcellulose (Chinese Patent Publication CN1203792A). The core tablet used for this tablet is obtained by wet granulation of a mixture containing povidone-iodine, citric acid, sugar powder, acacia and the like, and then compressing for tablet making by using the resulting granules. Further, Japanese Patent Application Unexamined Publication (Kokai) No. 63-77805 discloses a composition in the form of granule or powder consisting of povidone-iodine and at least one of urea or a sugar alcohol, which is easily dissolvable in water. This composition is dissolved before use and used as a gargle or the like, and not administered orally in the form of a tablet or the like.

### Disclosure of the Invention

### Object to be Solved by the Invention

An object of the present invention is to provide an orally disintegratable solid preparation comprising povidone-iodine. More specifically, the object of the present invention is to provide high quality solid preparation having a constant content of povidone-iodine per one unit of solid preparation.

### Means to Achieve the Object

In order to obtain a constant content of an active ingredient in a solid preparation, a method is generally employed which comprises the steps of performing wet granulation after the active ingredient is solved in a solvent, and then compressing the resulting granules. The inventors of the present invention at first considered that the aforementioned problem was solvable by employing wet granulation and conducted researches. However, they encountered a new problem in that a content of iodine per solid preparation was reduced due to the sublimation of iodine from the preparation during drying process for removing a solvent, and further manufacturing apparatuses were corroded by iodine sublimated from the preparation to cause a problem of degraded safety of workers. Furthermore, the inventors found that, for distribution of povidone-iodine in the whole oral cavity by using a solid preparation that is disintegratable and dissolvable in the oral cavity, it was necessary to prepare a solid preparation having a disintegrating time of about several minutes in the oral cavity, and also found that a solid preparation containing glucose, which is usually used as an excipient, had a problem of having too short disintegrating time to sufficiently distribute povidone-iodine in the oral cavity.

On the basis of these findings, the inventors of the present invention further conducted researches to solve the aforementioned new problem. As a result, they found that when a solid preparation is prepared by direct powder compression without applying a wet granulation step by using a granular sugar alcohol as an excipient and without addition of a basic ingredient, a constant content of povidone-iodine per 1 unit solid preparation was achieved, and moreover, an effective iodine was stably retained in the preparation. It was also found that, according to the production method of the present invention, the aforementioned problem resulting from the sublimation of iodine from a povidone-iodine preparation was completely avoidable, and further found that, in the solid preparation obtained as described above, povidone-iodine had an action similar to that of a binder, and thus a desirable disintegrating time in the oral cavity was successfully attained without adding an ordinarily used binder. The present invention was achieved on the basis of the aforementioned findings.

The present invention thus provides an orally disintegratable solid preparation comprising povidone-iodine as an active ingredient, which is obtained by direct powder compression of a mixture containing a granular sugar alcohol and povidone-iodine, wherein the mixture does not contain a basic ingredient and is not subjected to wet granulation. According to preferred embodiments of the present invention, there are provided the aforementioned solid preparation, wherein the aforementioned mixture does not contain a binder ordinarily used in the field of art; the aforementioned solid preparation, wherein the aforementioned mixture contains an acidic disintegrating agent, for example, alginic acid, carmellose or the like; the aforementioned solid preparation, wherein the aforementioned mixture contains a sweetener, for example, aspartame, acesulfame potassium or the like; the aforementioned solid preparation, which has a disintegrating time in oral cavity of about 1 to 10 minutes, preferably about 2 to 4 minutes; and the aforementioned solid preparation, which has a hardness of 40 to 200 N, preferably 80 to 120 N.

From another aspect, the present invention provides a method for producing an orally disintegratable solid preparation containing povidone-iodine as an active ingredient, which comprises the step of direct powder compression of a mixture containing a granular sugar alcohol and povidone-iodine without applying wet granulation, wherein the mixture does not contain a basic ingredient.

### Best Mode for Carrying out the Invention

The solid preparation of the present invention is an orally disintegratable-type solid preparation containing povidone-iodine as an active ingredient, and is obtained by directly compressing a mixture containing a granular sugar alcohol and povidone-iodine (provided that the mixture does not contain a basic ingredient) without applying wet granulation.

Povidone-iodine is a medicament described in the Japanese Pharmacopoeia, 14th Edition, and is a complex of a polymer of 1-vinyl-2-pyrrolidone (polyvinylpyrrolidone) and iodine. A content of iodine in povidone-iodine is usually about 9 to 12%. The povidone-iodine can be obtained as a commercial product (for example, "Povidone-iodine" produced by BASF Japan, "Povidone-iodine" produced by ISP Japan and the like). Particle sizes of crystals of povidone-iodine are not particularly limited. For example, crystals having a particle size of about 20 to 300 *µ* m can be used.

Types of the sugar alcohol used for the manufacture of the solid preparation of the present invention are not particularly limited. For example, D-sorbitol, D-mannitol and the like are preferred. As the sugar alcohol, a sugar alcohol fabricated in a granular form can be used. A particle size of the granules of the sugar alcohol may be, for example, about 50 to 1,500 *µ* m, preferably about 50 to 1,000 *µ* m. These types of sugar alcohols can be obtained as commercial products (for example, "NEOSORB P20/60" (mean particle diameter: 650 µ m), "NEOSORB P30/60" (mean particle diameter: 480 *µ*m), "NEOSORB P60W" (mean particle diameter: 180 µm), "NEOSORB P100T" (mean particle diameter: 110 µm), "PEARLITOL 100SD" (mean particle diameter: 100 µm), "PEARLITOL 200SD" (mean particle diameter: 200 µm) and the like which are all produced by Roquette). A ratio of the sugar alcohol and povidone-iodine is not particularly limited. In general, the sugar alcohol can be used in an amount so as to be sufficient to obtain a weight of about 300 to 1,000 mg, preferably 400 to 800 mg for a single administration unit of the solid preparation, and an amount of povidone-iodine can be controlled so that about 1 to 10 mg, preferably about 5 mg, of povidone-iodine is contained per single administration unit of the solid preparation. More specifically, it is desirable that the weight ratio of the sugar alcohol and povidone-iodine is about 200:1 to 60:1, preferably about 160:1 to 60:1.

For the manufacture of the solid preparation of the present invention, a disintegrating agent may be added to the aforementioned mixture. Although basic disintegrating agents have been generally used as disintegrating agents, it is desirable to use an acidic disintegrating agent because povidone-iodine is unstable under a basic condition. As a disintegrating agent as mentioned above, for example, carmellose, alginic acid and the like can be used. A ratio of the disintegrating agent in the mixture is not particularly limited. Where the weight of a single administration unit of the solid preparation is about 300 to 1,000 mg, preferably about 400 to 800 mg, the disintegrating agent may be added in an amount of about 10 to 120 mg, preferably about 30 to 60 mg. By using a sugar alcohol in an amount as mentioned above, a disintegrating time in the oral cavity of about several minutes can generally be obtained. Nevertheless, by using a disintegrating agent, it becomes possible to further control the disintegrating time. The solid preparation of the present invention preferably disintegrates in the oral cavity within several minutes, more preferably about 1 to 10 minutes, still more preferably about 2 to 4 minutes. It is desirable that the preparation contains the sugar alcohol and the disintegrating agent in combination so that the aforementioned disintegrating time can be attained. Further, the solid preparation of the present invention preferably has a hardness of 40 to 200 N, more preferably 80 to 120 N.

To the aforementioned mixture, pharmaceutical additives such as corrigents, sweeteners, stabilizers, and lubricants may be added in addition to the aforementioned ingredients. As the corrigents, L-menthol, caramel, various fruit perfumes and the like can be used. As the sweeteners, for example, aspartame, acesulfame potassium, dipotassium glycyrrhizinate, saccharin, saccharin sodium and the like can be used. As the stabilizers, for example, croscarmellose sodium, starch such as corn starch, rice starch and potato starch, and the like can be used. As the lubricants, for example, sodium stearoyl fumarate, magnesium stearate, calcium stearate, talc, sucrose esters of fatty acid and the like can be used. Each of these above pharmaceutical additives can be used in an amount of about 0.1 to 10% by weight, preferably about 1% by weight, based on the total weight of the aforementioned mixture.

The solid preparation of the present invention may contain one or more active ingredients having variety of pharmacological actions. Types of the active ingredient are not particularly limited. Examples include gambir, fennel, cetylpyridinium chloride, lysozyme chloride, yellow bark, licorice, platycodi radix, apricot kernel, potassium guaiacolsulfonate, dipotassium glycyrrhizinate, potassium cresolsulfonate, cassia bark, plantago seed, plantago herb, ginger, seneca snakeroot, mulberry bark, perilla herb, panax rhizome, citrus unshiu peel, ginseng, noscapine, ophiopogon tuber, peppermint, pinelliae tuber, chlorpheniramine maleate, menthol, eucalyptus, and the like.

The solid preparation of the present invention can be prepared by direct powder compression of a mixture obtained by mixing povidone-iodine and the aforementioned granular sugar alcohol. In the specification, the term "direct powder compression" means that compression molding (tablet making) of powder is carried out by directly compressing the powder without applying wet granulation. More specifically, the mixture containing povidone-iodine and granular sugar alcohol as well as the aforementioned arbitrarily used ingredients (disintegrating agent, corrigent, sweetener or the like) is prepared, and the compression step is directly performed by using the aforementioned mixture in the form of powder without a wet granulation step.

In the aforementioned mixture, it is not necessary to add a binder which is ordinarily used in the preparations of solid preparations such as tablets and the like manufactured by compression molding. Although it is not intended to be bound by any specific theory, in the mixture containing povidone-iodine at a ratio as mentioned above, povidone-iodine may exert binding action at an appropriate degree on the sugar alcohol, thereby the direct powder tablet making by compression molding becomes possible without using an ordinarily used binder. For the manufacture of troches, a step of compression is generally applied to granules obtained by wet granulation using a solvent such as ethanol and water after the addition of 3 to 5% by weight of a binder. According to the method of the present invention, by direct compression of the aforementioned mixture without using wet granulation, a solid preparation can be obtained in a very convenient manner, and moreover, the resulting preparation has a desirable disintegrating time in the oral cavity.

A form of the orally disintegratable solid preparation of the present invention is not particularly limited. A form of a tablet is generally preferred and examples include troches and the like. The solid preparation of the present invention is a preparation to be retained in the oral cavity for several minutes. Accordingly, the preparation may have a form of tablet having a hole in the center to prevent asphyxiation accident at aspiration of the preparation in the trachea.

According to the solid preparation of the present invention, povidone-iodine can be distributed in the whole oral cavity and pharynx (epipharynx, oropharynx, and hypopharynx) by keeping about 1 or 2 unit dosage forms in the oral cavity per single administration, and allowing the preparation disintegrated in several minutes under contact with saliva. The preparation is especially effective for amelioration of a pain due to inflammation of the throat with common cold, sterilization and disinfection of the oral cavity and throat with tonsillitis and the like. A dose of the solid preparation of the present invention is not particularly limited. The dose may be about 5 to 50 mg per day as the weight of povidone-iodine.

### Examples

The present invention will be explained more specifically with reference to the following examples. However, the scope of the present invention is not limited by the following examples.

### Example 1

Povidone-iodine (povidone-iodine according to Japanese Pharmacopoeia, produced by BASF Japan, 10 g), carmellose (NS-300, produced by NICHIRIN CHEMICAL INDUSTRIES, LTD., 40 g), Aspartame (Aspartame, produced by Ajinomoto Co., Inc., 8 g), D-sorbitol (NEOSORB P60W, produced by Roquette, 726 g), 20% L-menthol powder (8 g) and sodium stearoyl fumarate (Pruv, produced by Kimura Sangyo Co., Ltd., 8 g) were mixed, and troches having a weight of 400 mg per troche were prepared by using a compressing machine. The troches had a tablet hardness of 104 N.

The 20% L-menthol powder mentioned above is a mixture of L-menthol (1-menthol, produced by TAKASAGO INTERNATIONAL CORP.) and hydrous silicon dioxide (Adsolider 102, produced by Freund Corporation) at a ratio of 1 to 4, and the same powder was used was used in the following examples and comparative examples.

### Example 2

Povidone-iodine (povidone-iodine according to Japanese Pharmacopoeia, produced by BASF Japan, 10 g), carmellose (NS-300, produced by NICHIRIN CHEMICAL INDUSTRIES, LTD., 60 g), acesulfame potassium (Sunett, produced by Nutrinova, 16 g), D-sorbitol (NEOSORB P60W, produced by Roquette, 698 g), 20% L-menthol powder (8 g) and magnesium stearate (Magnesium Stearate, produced by TAIHEI CHEMICAL INDUSTRIAL CO., LTD., 8 g) were mixed and used to prepare troches having a weight of 400 mg per troche by using a compressing machine. The troches had a tablet hardness of 118 N.

### Comparative Example 1

Povidone-iodine (povidone-iodine according to Japanese Pharmacopoeia, produced by BASF Japan, 10 g), croscarmellose sodium (Kiccolate ND-2HS, produced by Asahi Kasei Chemicals, 40 g), Aspartame (Aspartame, produced by Ajinomoto Co., Inc., 8 g), glucose (Glucose Crista A, produced by SANMATSU KOGYO CO., LTD., 726 g), 20% L-menthol powder (8 g) and sodium stearoyl fumarate (Pruv, produced by Kimura Sangyo Co., Ltd., 8 g) were mixed and used to prepare troches having a weight of 400 mg per troche by using a compressing machine. The troches had a tablet hardness of 43 N.

### Comparative Example 2

Povidone-iodine (povidone-iodine according to Japanese Pharmacopoeia, produced by BASF Japan, 10 g), carmellose (NS-300, produced by NICHIRIN CHEMICAL INDUSTRIES, LTD., 40 g), Aspartame (Aspartame, produced by Ajinomoto Co., Inc., 8 g), D-sorbitol (NEOSORB P60W, produced by Roquette, 706 g), 20% L-menthol powder (8 g), sodium stearoyl fumarate (Pruv, produced by Kimura Sangyo Co., Ltd., 8 g) and magnesium aluminometasilicate (Neusilin S2, produced by Fuji Chemical Industry Co., Ltd., 20 g) were mixed and used to prepare troches having a weight of 400 mg per troche by using a compressing machine. The troches had a tablet hardness of 87 N.

### Comparative Example 3

Povidone-iodine (povidone-iodine according to Japanese Pharmacopoeia, produced by BASF Japan, 10 g), carmellose (NS-300, produced by NICHIRIN CHEMICAL INDUSTRIES, LTD., 40 g), Aspartame (Aspartame, produced by Ajinomoto Co., Inc., 8 g) and D-sorbitol (NEOSORB P60W, produced by Roquette, 726 g) were mixed, then added with 80% alcohol (250 mL), granulated, dried and sized to obtain granules. These granules, 20% L-menthol powder (8 g) and sodium stearoyl fumarate (Pruv, produced by Kimura Sangyo Co., Ltd., 8 g) were mixed and used to prepare troches having a weight of 400 mg per troche by using a compressing machine. The troches had a tablet hardness of 95 N.

### Test Example 1: Measurement of effective iodine content

Effective iodine contents of the solid preparations of the examples and the comparative examples were measured immediately after the manufacture and after storage of 1 week at 60°C (form of package: bottled with stopper) by the neutralization titration method using a sodium thiosulfate solution, and remaining ratios were calculated. The results are shown in Table 1.

**Table 1**

| | Example 1 | Example 2 | Comparative Example 1 | Comparative Example 2 | Comparative Example 3 |
|---|---|---|---|---|---|
| Povidone-iodine | 5 | 5 | 5 | 5 | 5 |
| Carmellose | 20 | 30 | - | 20 | 20 |
| Croscarmellose sodium | - | - | 20 | - | - |
| Aspartame | 4 | - | 4 | 4 | 4 |
| Acesulfame potassium | - | 8 | - | - | - |
| D-Sorbitol | 363 | 349 | - | 353 | 363 |
| Glucose | - | - | 363 | - | - |
| Magnesium aluminometasilicate | - | - | - | 10 | - |
| 20% L-Menthol powder | 4 | 4 | 4 | 4 | 4 |
| Sodium stearoyl fumarate | 4 | - | 4 | 4 | 4 |
| Magnesium stearate | - | 4 | - | - | - |
| Total (mg/T) | 400 | 400 | 400 | 400 | 400 |
| Immediately after manufacture (%) | 100 | 100 | 100 | 100 | 90 |
| 60°C, 1 week (%) | 70 | 70 | 0 | 0 | 55 |

As shown in Table 1, the preparation of Comparative Example 1 which used glucose instead of D-sorbitol as a sugar alcohol, and the preparation of Comparative Example 2 which was added with magnesium aluminometasilicate as a basic ingredient were found to be preparations having extremely poor stability because they gave remaining ratios of 0 after the storage for 1 week at 60°C, although they had no problem immediately after the manufacture. The preparation of Comparative Example 3, which was prepared through wet granulation, gave 10% reduction of the content even immediately after the manufacture, and also gave a remaining ratio of 55% after the storage for 1 week at 60°C, which results were not satisfactory.

Whilst, the preparations of Examples 1 and 2 according to the present invention maintained 70% of remaining ratio even after the storage for 1 week at 60°C, and thus they were found to have excellent stability.

### Test Example 2: Uniformity of povidone-iodine content

Effective iodine contents were measured for each of the preparations of Examples 1 and 2 (each 10 tablets). Relative standard deviations were calculated to be 1.9% and 1.6%, respectively, and accordingly no variation in the effective iodine content was observed.

### Test Example 3: Measurement of disintegrating time in oral cavity

One tablet of each of the solid preparations of Example 1 and Comparative Example 1 was taken into the mouth by a panel consisting of 10 persons without using water, and time required for disintegration of the preparation in the oral cavity was measured. Average of the times for 10 persons was considered as intraoral disintegrating time. The results are shown in Table 2.

### Test Example 4: Ingestibility

At the time of the measurement of the intraoral disintegrating time mentioned above, ingestibility of the tablets including taste and mouthfeel was evaluated. Taste was evaluated according to 5-stage criteria: good, fairly good, moderate, slightly bad, and bad. Mouthfeel was evaluated according to 3-stage criteria: no roughness, slight roughness, and roughness. The results are shown in Table 2.

**Table 2**

| | | Example 1 | Comparative Example 1 |
|---|---|---|---|
| Intraoral disintegrating time (minute) | | 3.2 | 0.5 |
| Taste | Good | 8 persons | 0 |
| | Fairly good | 2 persons | 0 |
| | Moderate | 0 | 1 person |
| | Slightly bad | 0 | 5 persons |
| | Bad | 0 | 4 persons |
| Mouthfeel | No roughness | 10 persons | 1 person |
| | Slight roughness | 0 | 2 persons |
| | Roughness | 0 | 7 persons |

The intraoral disintegrating time of the solid preparation of Example 1 was 3.2 minutes, which guarantees a time required for povidone-iodine having the high sterilizing property to exert sufficient bactericidal effect in the whole oral cavity and pharynx (usually 1 minutes or longer), and the preparation also gave superior evaluation results for taste and mouthfeel. Whilst, the intraoral disintegrating time of the solid preparation of Comparative Example 1 was found to be 0.5 minute, and the preparation was completely dissolved and disintegrated in the mouth before povidone-iodine was sufficiently distributed in the oral cavity. Therefore, the preparation failed to give sufficient effect in the whole oral cavity. Moreover, the preparation was disfavored for taste and mouthfeel.

### Industrial Applicability

The solid preparation of the present invention is a solid preparation having a constant content of povidone-iodine per a single unit solid preparation, and has high stability of effective iodine. The preparation has features that it can be very conveniently and efficiently produced by direct powder compression without causing problems of corrosion of manufacturing apparatuses and aggravation of working environment due to sublimation of iodine generated from the preparation during wet granulation. Moreover, the solid preparation of the present invention can disintegrate within about 2 to 4 minutes to sufficiently distribute povidone-iodine in the oral cavity when kept orally, and therefore, the preparation achieves extremely convenient disinfection of the oral cavity and pharynx.

## Claims

1. An orally disintegrable solid preparation comprising povidone-iodine as an active ingredient, which is obtained by direct powder compression of a mixture containing a granular sugar alcohol and povidone-iodine, wherein the mixture does not contain a basic ingredient and is not subjected to wet granulation.

2. The solid preparation according to claim 1, wherein the mixture contains a disintegrating agent selected from the group consisting of alginic acid and carmellose.

3. The solid preparation according to claim 1 or 2, wherein the mixture further contains a sweetener selected from the group consisting of Aspartame and acesulfame potassium.

4. The solid preparation according to any one of claims 1 to 3, which has an intraoral disintegrating time of about 1 to 10 minutes.

5. A method for manufacturing an orally disintegratable solid preparation comprising povidone-iodine as an active ingredient, which comprises the step of direct powder compression of a mixture containing a granular sugar alcohol and povidone-iodine without applying wet granulation, wherein the mixture does not contain a basic ingredient.
